(19)

Europäisches
Patentamt
European
Patent Office
Office européen
des brevets

(11) **EP 2 106 733 A1**

(12) **EUROPEAN PATENT APPLICATION**
published in accordance with Art. 153(4) EPC

(43) Date of publication:
**07.10.2009 Bulletin 2009/41**

(51) Int Cl.:
***A61B 1/00*** (2006.01)

(21) Application number: **08704203.2**

(22) Date of filing: **30.01.2008**

(86) International application number:
**PCT/JP2008/051440**

(87) International publication number:
**WO 2008/093745 (07.08.2008 Gazette 2008/32)**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **31.01.2007 JP 2007021558**

(71) Applicant: **Olympus Corporation**
**Tokyo 151-0072 (JP)**

(72) Inventor: **MORISHITA, Koki**
**Tokyo 151-0072 (JP)**

(74) Representative: **von Hellfeld, Axel et al**
**Wuesthoff & Wuesthoff**
**Schweigerstrasse 2**
**81541 München (DE)**

(54) **FLUORESCENCE OBSERVATION DEVICE FOR ORGANISM TISSUE**

(57)     The concentration distribution of a fluorescence dye capable of selectively staining normal tissue and abnormal tissue is accurately observed by eliminating the unevenness due to irregular distance from the distal end of an endoscope to an organism surface or the surface roughness of the organism. There is provided a fluorescence observation device for organism tissue comprising: an excitation light optical system for irradiating an organism tissue adhered or infiltrated with a first fluorescence dye whose stainability is different between normal tissue and abnormal tissue in the organism tissue, and a second fluorescence dye whose fluorescence wavelength or absorption wavelength is different from that of the first fluorescence dye and whose stainability is nonselective between normal tissue and abnormal tissue in the organism tissue, with excitation light which excites the first fluorescence dye and the second fluorescence dye, either simultaneously or in a time sharing manner; a fluorescence detecting section for separately detecting fluorescence from the first fluorescence dye and fluorescence from the second fluorescence dye excited by the excitation light from the excitation light optical system; a compensation processing section for compensating the fluorescence information from the first fluorescence dye, based on the fluorescence information from the second fluorescence dye detected by the fluorescence detecting section; and a display section for displaying the fluorescence information compensated by the compensation processing section.

FIG. 1

## Description

Technical Field

The present invention relates to a fluorescence observation device for organism tissue.

Background Art

It is known that abnormal tissue such as cancer tissue and other normal tissue are different in the amounts of expressed molecules, cell activities, and the like. There have been proposed methods for endoscopically discriminating cancer through observation of such difference by using a fluorescent probe (fluorescence dye) as the difference in the fluorescence intensity (for example, see Patent Document 1 and Patent Document 2).

> Patent Document 1:
> Japanese Unexamined Patent Application, Publication No. Hei 10-201707
> Patent Document 2:
> Japanese Unexamined Patent Application, Publication No. Hei 6-27110

Disclosure of Invention

In Patent Document 1 and Patent Document 2 have been disclosed an endoscope for diagnosis through observation of a difference between abnormal tissue and normal tissue by using one type of fluorescent probe (difference in the accumulation of the fluorescent probe).
It is also known that some fluorescent probes are accumulated in tumor or inflammatory sites to emit fluorescence therefrom. In addition, since an organism tissue naturally emits its autofluorescence, it is difficult to exclusively observe the fluorescence originated from tumor sites, no matter how excellent cancer diagnosing probe has been developed. Therefore, upon diagnosis on whether the tissue is normal or abnormal by using a fluorescent probe, it is desirably possible to compare the fluorescence intensity between normal and abnormal tissues.

However, with an observation apparatus such as an endoscope, since the distance from the distal end of the endoscope to the organism tissue is irregular and the surface of the organism tissue is rough, then the distance from the distal end of the endoscope and the angle of irradiation of excitation light on the organism surface vary depending on the location. For this reason, inconveniently, the intensity of excitation light irradiated on the organism surface differs depending on the location. Furthermore, since the distance from the distal end of the endoscope to the organism surface and the angle of the organism surface vary depending on the location, then, inconveniently, emitted fluorescence is each differently attenuated until reaching the distal end of the endoscope, or other inconveniences may be brought about.

As a result, even though the fluorescent probe is accumulated at the same concentration, the observed fluorescence intensity differs depending on the location, which makes it difficult to accurately diagnose whether the tissue is normal or abnormal on the basis of the detected fluorescence intensity. In order to improve the diagnosis accuracy, fluorescence has to be detected over a plurality of times by changing the position and the angle of the distal end of the endoscope, and therefore, problematically, the examination takes time.

The present invention takes the above situations into considerations with an object of providing a fluorescence observation device for organism tissue with which the concentration distribution of a fluorescence dye capable of selectively staining normal tissue and abnormal tissue can be accurately observed by eliminating the unevenness due to irregular distance to the organism surface or the surface roughness of the organism.

In order to achieve the above object, the present invention provides the following solutions.
One aspect of the fluorescence observation device for organism tissue according to the present invention comprises: an excitation light optical system for irradiating an organism tissue adhered or infiltrated with a first fluorescence dye whose stainability is different between normal tissue and abnormal tissue in the organism tissue, and a second fluorescence dye whose fluorescence wavelength or absorption wavelength is different from that of the first fluorescence dye and whose stainability is nonselective between normal tissue and abnormal tissue in the organism tissue, with excitation light which excites the first fluorescence dye and the second fluorescence dye, either simultaneously or in a time sharing manner; a fluorescence detecting section for separately detecting fluorescence from the first fluorescence dye and fluorescence from the second fluorescence dye excited by the excitation light from the excitation light optical system; a compensation processing section for compensating the fluorescence information from the first fluorescence dye, based on the fluorescence information from the second fluorescence dye detected by the fluorescence detecting section; and a display section for displaying the fluorescence information compensated by the compensation processing section.

In the above aspect, the second fluorescence dye may be cationic.
In addition, the above aspect, the second fluorescence dye may be lipophilic.

Moreover, in the above aspect, the compensation processing section may normalize the fluorescence information from the first fluorescence dye that has been detected by the fluorescence detecting section, by the fluorescence information from the second fluorescence dye.
Furthermore, in the above aspect, the fluorescence detecting section may also comprise a single observation optical system, whose relative position with respect to the excitation light optical system is fixed, for guiding both fluorescences from the first fluorescence dye and the second fluorescence dye toward the fluorescence detecting section.

The present invention demonstrates an effect in which the concentration distribution of a fluorescence dye capable of selectively staining normal tissue and abnormal tissue can be accurately observed through fluorescence observation by eliminating the unevenness of the intensity distribution of excitation light on the organism surface due to irregular distance to the organism surface or the surface roughness of the organism.

Brief Description of Drawings

[FIG. 1] FIG. 1 is a block diagram showing the overall structure of an endoscope system comprising a fluorescence observation device for organism tissues according to a first embodiment of the present invention.

[FIG. 2] FIG. 2 is a schematic block diagram showing the structure inside the imaging unit of the endoscope system of FIG. 1.

[FIG. 3] FIG. 3 shows the transmittance characteristics of optical components which constitute the endoscope system of FIG. 1, and the wavelength characteristics of illumination lights and fluorescences.

[FIG. 4] FIG. 4 is a block diagram showing the inner structure of the image storing section of the endoscope system of FIG. 1.

[FIG. 5] FIG. 5 is a timing chart illustrating the operation during the white light observation of the endoscope system of FIG. 1.

[FIG. 6] FIG. 6 is a timing chart illustrating the operation during the fluorescence observation of the endoscope system of FIG. 1.

[FIG. 7] FIG. 7 is a timing chart showing an example of the observation operation of the endoscope system of FIG. 1.

[FIG. 8A] FIG. 8A is a schematic diagram illustrating the observation status of the endoscope system of FIG. 1.

[FIG. 8B] FIG. 8B is a graph showing the excitation light intensity distribution by each region in the observation with the endoscope system of FIG. 1.

[FIG. 8C] FIG. 8C is a graph showing the dye distribution by each region in the observation with the endoscope system of FIG. 1.

[FIG. 8D] FIG. 8D is a graph showing the fluorescence intensity distribution by each region in the observation with the endoscope system of FIG. 1.

[FIG. 8E] FIG. 8E is a graph showing the fluorescence intensity distribution by each region in the observation with the endoscope system of FIG. 1.

[FIG. 8F] FIG. 8F is a graph showing the dye distribution (corrected fluorescence intensity distribution) by each region in the observation with the endoscope system of FIG. 1.

[FIG. 9A] FIG. 9A is a microphotograph showing the fluorescence image of an organism tissue stained with a first fluorescence dye alone.

[FIG. 9B] FIG. 9B is a schematic diagram illustrating the microphotograph of FIG. 9A.

[FIG. 10] FIG. 10 is a microphotograph showing the fluorescence image of the same organism tissue as that of FIG. 9A stained with a second fluorescence dye alone.

[FIG. 11] FIG. 11 shows a resultant image after the fluorescence intensity distribution of FIG. 9A has been divided by the fluorescence intensity distribution of FIG. 10.

[FIG. 12] FIG. 12 is a timing chart showing a modified example of the observation operation of FIG. 7.

[FIG. 13] FIG. 13 is a timing chart showing another modified example of the observation operation of FIG. 7.

Explanation of Reference Signs:

A: observation target (organism tissue)

Ga and Gb: fluorescence image information of fluorescence dye

Gc: reflected light image (image which reflects the blood volume)

1: endoscope system (fluorescence observation device for organism tissue)

3: imaging unit (fluorescence detecting section: observation optical system)

6: display unit (display section)

7: light guide (excitation light optical system: observation optical system)

8: white observation light source

9: special observation light source (excitation light optical system)

13: variable spectral element

17: image storing section

18: image processing section (compensation processing section)

Best Mode for Carrying Out the Invention

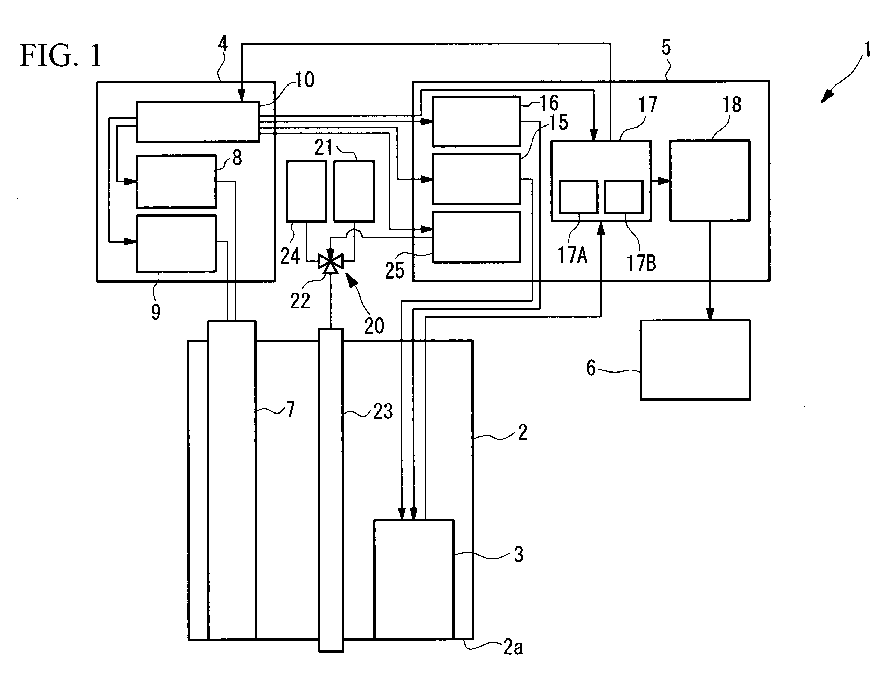

The fluorescence observation device for organism tissue according to this embodiment is equipped in an endoscope system 1. As shown in FIG. 1, the endoscope system 1 comprises an insertion section 2 to be inserted into a body cavity of an organism, an imaging unit 3 disposed in the insertion section 2, a light source unit 4 for emitting a plurality of types of lights, a liquid delivery unit 20 for supplying a liquid to be discharged from the distal end 2a of the insertion section 2, a control unit 5 for controlling the imaging unit 3, the light source unit 4, and the liquid delivery unit 20, and a display unit 6 for displaying the image captured by the imaging unit 3.

In addition, the fluorescence observation device for organism tissue according to this embodiment comprises the imaging unit 3, the light source unit 4, the control unit 5, and the display unit 6.

The insertion section 2 has an extremely narrow outer dimension to be insertable into a body cavity of an organism, and comprises therein a light guide 7 for transmitting lights from the imaging unit 3 and the light source unit 4 to the distal end 2a.

The light source unit 4 comprises: a white observation light source 8 for emitting illumination light which illuminates an imaging target (observation target) A in the body cavity so as to capture its white image; a special observation light source 9 for emitting excitation light to be irradiated on the imaging target A in the body cavity so as to excite a fluorescent substance existing in the imaging target A to thereby generate fluorescence, and illumination light having a wavelength highly absorbable in blood vessels; and a light source controlling circuit 10 for controlling these light sources 8 and 9.

The white observation light source 8 is, for example, a combination of a xenon lamp (not shown) and a bandpass filter. The bandwidth at half maximum transmission of the bandpass filter is not less than 420 nm and not more than 470 nm, not less than 500 nm and not more than 580 nm, and not less than 570 nm and not more than 650 nm. That is to say, the white observation light source 8 is capable of emitting blue illumination light having a wavelength band of not less than 420 nm and not more than 470 nm, green illumination light having a wavelength band of not less than 500 nm and not more than 580 nm, and red illumination light having a wavelength band of not less than 570 nm and not more than 650 nm.

The special observation light source 9 is, for example, a combination of three laser light sources (not shown): a first semiconductor laser for emitting first excitation light having a peak wavelength of $490 \pm 5$ nm (or an argon laser for emitting excitation light of $488 \pm 5$ nm), a second semiconductor laser for emitting second excitation light having a peak wavelength of $780 \pm 5$ nm, and a third semiconductor laser for emitting illumination light having a peak wavelength of $420 \pm 5$ nm.

The first semiconductor laser is capable of exciting, for example, a first fluorescence dye which has a fluorescein structure. Moreover, the second semiconductor laser is capable of exciting a second fluorescence dye which has a tricarbocyanine structure. Furthermore, the third semiconductor laser emits light having a wavelength highly absorbable in blood, and thus is capable of contrast radiography of blood vessels through observation of the reflected light.

As for the first fluorescence dye, for example, an esterase-sensitive fluorescent probe having a fluorescein structure is used. The esterase-sensitive fluorescent probe will not generate fluorescence until it reacts with esterase. However, it has a property of changing into a substance which has a photoabsorption peak around 490 nm and generates fluorescence having a peak around 510 nm, with the presence of esterase which is a cytoplasmic hydrolase.

The amount of the existing esterase is more abundant in cancer tissue than in normal tissue. Therefore, the esterase changes into fluorescein more rapidly in cancer tissue. Accordingly, more intense fluorescence can be captured from cancer tissue than from normal tissue by irradiating the first excitation light from the first laser light source.

The second fluorescence dye is, for example, a lipophilic and cationic fluorescence of a tricarbocyanine structure having a fluorescence peak wavelength in a near infrared area, and generates fluorescence by irradiation of the second excitation light from the second laser light source. In addition, the second fluorescence dye is cationic and thus has an adsorptive property to the phospholipid, which is anionic, in the cytoplasmic membrane. Because of its lipophilicity, the second fluorescence dye is apt to exist in the cytoplasmic membrane. A mixture of these first fluorescence dye and second fluorescence dye is stored in a tank 21 that will be described later.

The light source controlling circuit 10 is to operate the white observation light source 8 and the special observation light source 9 at predetermined timings according to a timing chart that will be described later.

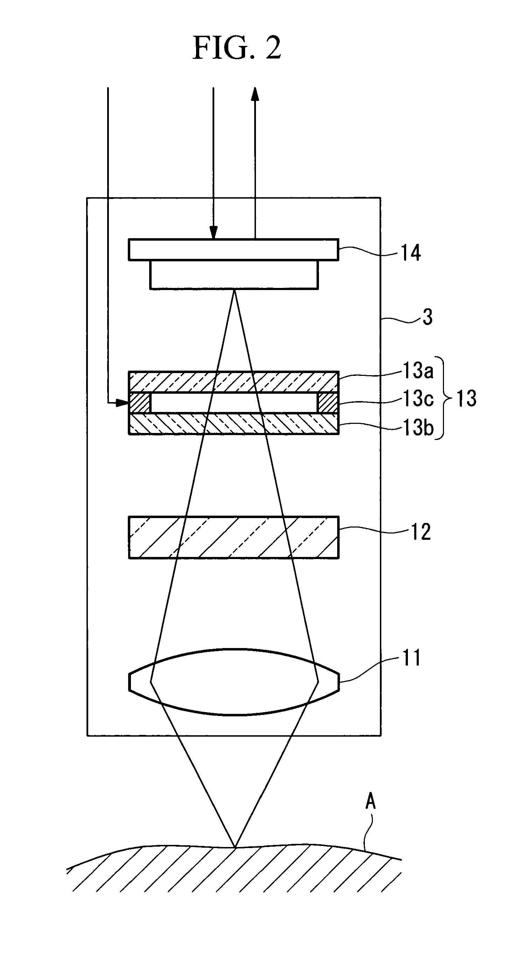

As shown in FIG. 2, the imaging unit 3 comprises an imaging optical system 11 for condensing light being incident from the imaging target A, an excitation light cut filter 12 for blocking excitation light being incident from the imaging target A, a variable spectral element 13 capable of changing the spectral characteristic by the operation of the control unit 5, and an imaging device 14 for capturing light that has been condensed by the imaging optical system 11 and converting the light into an electric signal.

The variable spectral element 13 is an etalon type optical filter comprising: two planer optical members 13a and 13b arranged with a parallel spacing therebetween and reflection films on the opposite surfaces thereof; and an actuator 13c for changing the spacing between these optical members 13a and 13b. The actuator 13c is, for example, a piezo device. This variable spectral element 13 is capable of changing the spacing dimension between the optical members 13a and 13b by the operation of the actuator 13c to thereby change the wavelength band of light which transmits therethrough.

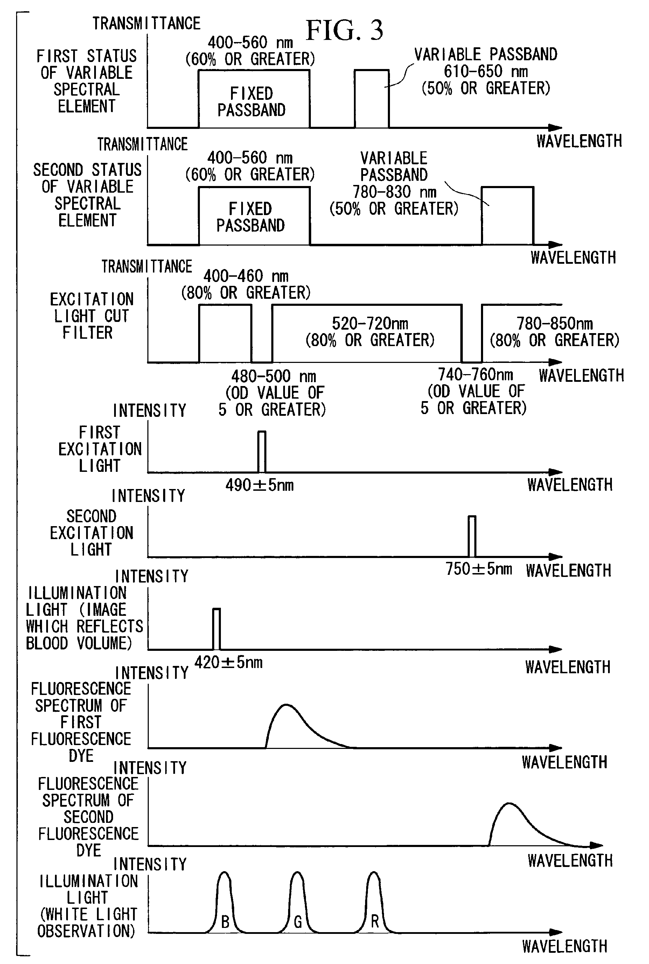

More specifically, as shown in FIG. 3, the variable spectral element 13 has a transmittance-wavelength characteristic having two passbands: one fixed passband and one variable passband. The fixed passband is to transmit incident light at all times irrespective of the status of the variable spectral element 13. In addition, the variable passband is to change the transmittance characteristic according to the status of the variable spectral element 13.

In this embodiment, the variable spectral element 13 has a variable passband in a red wavelength band (for example, not less than 610 nm and not more than 650 nm). In addition, the variable spectral element 13 is changed between two statuses according to the control signal from the control unit 5.

The first status is to increase the transmittance of 610-650 nm by 50% or greater to thereby allow light in a red (R) wavelength band to pass through.

The second status is to sufficiently reduce the transmittance of 610-650 nm as compared to that of the first status and to block light in a wavelength band of 780-830 nm from passing through.

The fixed passband is arranged within a range of not less than 400 nm and not more than 560 nm, and is fixed to have, for example, a transmittance of 60% or greater. By so doing, reflected lights of blue (B) and green (G) illumination lights, fluorescence from the first fluorescence dye generated by the irradiation of first excitation light from the first semiconductor laser, and reflected light caused by the irradiation of illumination light from the third semiconductor laser can be transmitted.

Accordingly, if arranged in the first status, the variable spectral element 13 can transmit reflected lights of blue, green, and red illumination lights, and fluorescence from the first fluorescence dye. Moreover, if arranged in the second status, the variable spectral element 13 can transmit reflected lights of blue and green illumination lights, fluorescences from the first and second fluorescence dyes, and reflected light caused by the irradiation of illumination light from the third semiconductor laser.

In addition, the excitation light cut filter 12 has a transmittance of 80% or greater in a wavelength band of 400-460 nm, an OD value of 5 or greater (= transmittance of $1 \times 10^{-5}$ or lower) in a wavelength band of 480-500 nm, a transmittance of 80% or greater in a wavelength band of 520-720 nm, an OD value of 5 or greater in a wavelength band of 740-760 nm, and a transmittance of 80% or greater in a wavelength band of 780-850 nm.

As shown in FIG. 1, the control unit 5 comprises an imaging device driving circuit 15 for drive-controlling the imaging device 14, a variable spectral element driving circuit 16 for drive-controlling the variable spectral element 13, a valve driving circuit 25 that will be described later, an image storing section 17 for storing image information acquired by the imaging device 14, and an image processing circuit (image processing section) 18 for processing the image information stored in the image storing section 17 and outputting the processed image information to the display unit 6.

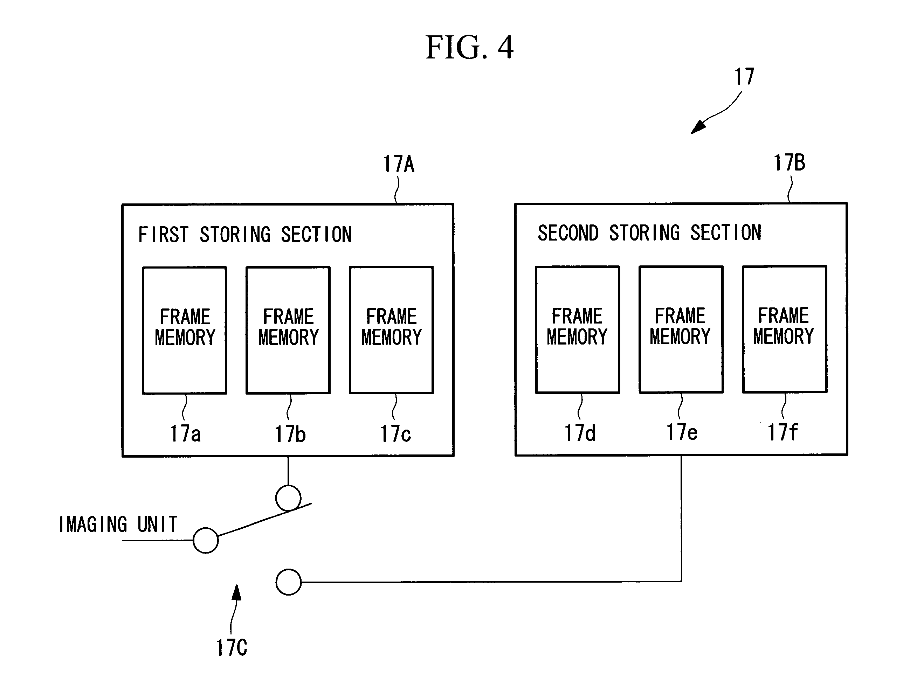

As shown in FIG. 4, the image storing section 17 comprises a first storing section 17A, a second storing section 17B, and a switch 17C for switching the input destination of the image information that has been output from the imaging device 14, between the first storing section 17A and the second storing section 17B. The first storing section 17A comprises frame memories 17a and 17b for storing pieces of fluorescence image information, and a frame memory 17c for storing image information which reflects the blood volume.

The second storing section 17B comprises a plurality of frame memories 17d to 17f for storing pieces of white light observation image information.

Moreover, the frame memory 17a stores the fluorescence image information Ga acquired by irradiating the first excitation light after the fluorescence dye has been sprayed, and by having the variable spectral element 13 in the first status. This fluorescence image information Ga serves as the fluorescence image information acquired by capturing fluorescence generated from the first fluorescence dye.

Furthermore, the frame memory 17b stores the fluorescence image information Gb acquired by irradiating the second excitation light after the fluorescence dye has been sprayed, and by having the variable spectral element 13 in the second status. This fluorescence image information Gb serves as the fluorescence image information acquired by capturing fluorescence generated from the second fluorescence dye.

In addition, the frame memory 17c stores the reflected light image (image which reflects the blood volume) Gc acquired by having the variable spectral element 13 in the first status, and by irradiating illumination light from the third semiconductor laser.

The wavelength band of this illumination light at 420 nm includes the photoabsorption band of hemoglobin. Therefore, by capturing the reflected light thereof, the image information which reflects the blood volume such as the structure of blood vessels relatively near the surface of the organism tissue can be acquired.

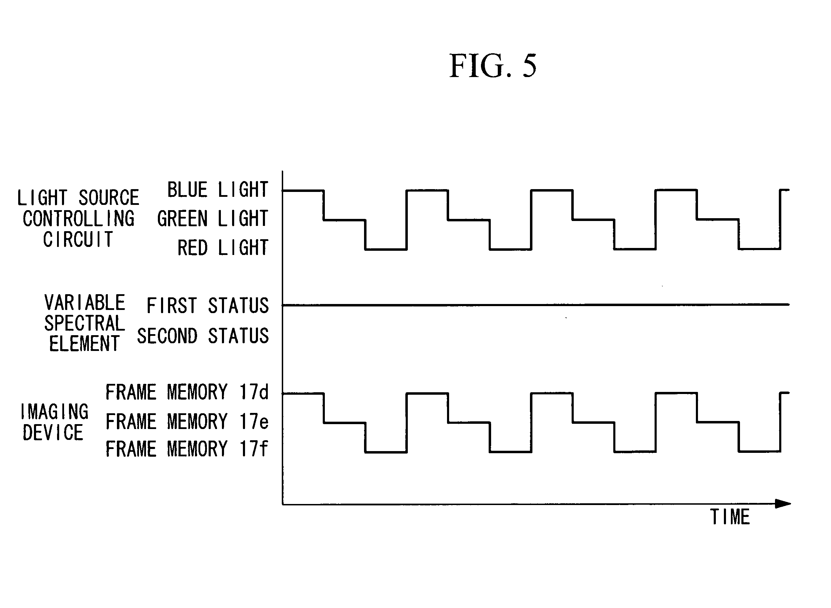

As shown in FIG. 5, the frame memories 17d to 17f of the second storing section 17B respectively store pieces of reflected light image information acquired by having the variable spectral element 13 in the first status, and by respectively irradiating blue light, green light, and red light within the reflected light image for the white light observation.

The imaging device driving circuit 15 and the variable spectral element driving circuit 16 are connected to the light source controlling circuit 10 so as to drive-control the variable spectral element 13 and the imaging device 14, synchronously with the switching operation between the white observation light source 8 and the special observation light source 9 done by the light source controlling circuit 10.

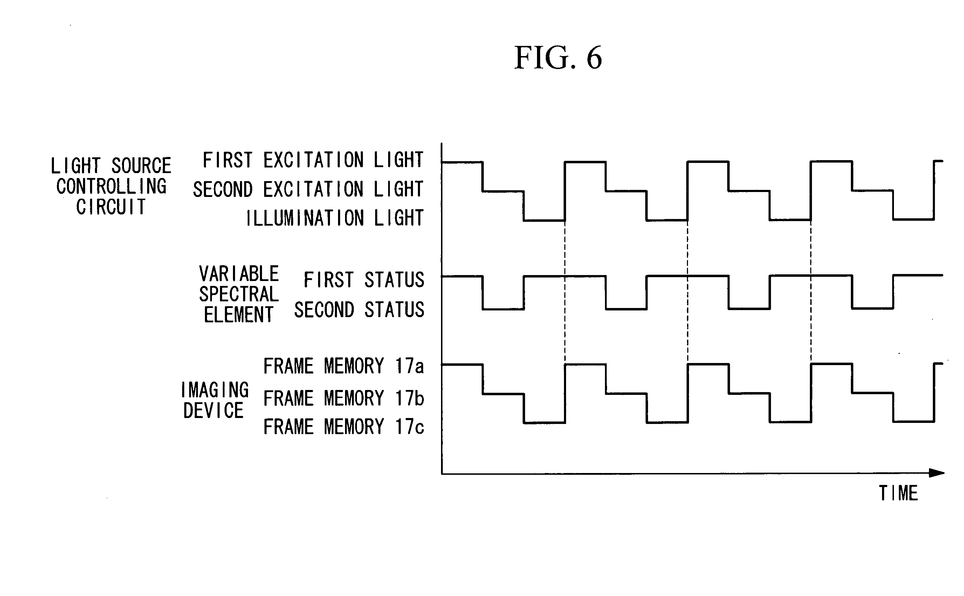

Specifically, as shown in the timing chart of FIG. 6, when the first excitation light, the second excitation light, or the illumination light from the third semiconductor laser, is emitted from the special observation light source 9 by the operation of the light source controlling circuit 10, the variable spectral element driving circuit 16 switches the status of the variable spectral element 13 to the first status or the second status. Moreover, together with this operation, the switch 17C is switched to the first storing section 17A side so that the image information output from the imaging device 14 by the imaging device driving circuit 15 can be output to the first storing section 17A.

In addition, when illumination light is emitted from the white observation light source 8 by the operation of the light source controlling circuit 10, the variable spectral element driving circuit 16 switches the status of the variable spectral element 13 to the first status, and the switch 17C is switched to the second storing section 17B side so that the image information output from the imaging device 14 by the imaging device driving circuit 15 can be output to the second storing section 17B.

Moreover, upon the white light observation, the image processing circuit 18 receives the pieces of reflected light image information acquired by irradiation of blue, green, and red illumination lights from the respective frame memories 17d to 17f of the second storing section 17B, and outputs these pieces of image information to the three R, G, and B channels of the display unit 6. Furthermore, upon the fluorescence observation, the image processing circuit 18 receives the pieces of fluorescence image information acquired by irradiation of excitation lights from the first storing section 17A, and applies the following inter-picture calculation to these pieces of image information.

$$G1 = \alpha \times Ga/Gb$$

Here, $\alpha$ denotes an arbitrary constant, and can be changed by an operation unit (not shown).

The fluorescence image information G1 is acquired by dividing the fluorescence image Ga of the first fluorescence dye by the fluorescence image Gb of the second fluorescence dye, and therefore shows the corrected (normalized) image of the fluorescence image of the first fluorescence dye by using the fluorescence intensity distribution of the second fluorescence dye.

Then, the image processing circuit 18 outputs, for example, the fluorescence image information G1 and the fluorescence image information Gb acquired by the irradiation of the excitation lights, and the reflected light image Gc acquired by the irradiation of the illumination light from the third semiconductor laser, respectively to the three R, G, and B channels of the display unit 6.

The liquid delivery unit 20 comprises a tank 21 for storing a fluorescent agent, a tank 24 for storing a washing liquid, a valve 22 for supplying/stopping such solutions from these tanks 21 and 24, a liquid delivery tube 23 connected to the valve 22 for supplying the solutions along the insertion section 2 to the distal end 2a, and a valve driving circuit 25 disposed in the control unit 5 for controlling the valve 22. The liquid delivery tube 23 has its distal end disposed in the distal end 2a of the insertion section 2 so that the delivered fluorescent probe can be sprayed towards the imaging target A. As for the liquid delivery tube 23, a forceps channel provided in the insertion section 2 may be utilized, too. In addition, the liquid delivery tube 23 and the valve 22 may also be respectively arranged for each of the tanks 21 and 24.

The valve driving circuit 25 controls the valve 22 so that a mixture of the first fluorescence dye and the second fluorescence dye which has been stored over a predetermined time in the tank 21 can be sprayed during the white light observation before the special observation light source 9 for acquiring the fluorescence image of the fluorescence dyes is started.

In addition, the valve driving circuit 25 switches the valve 22 to the off status after the fluorescence dye mixture has been sprayed.

Moreover, the valve driving circuit 25 controls the valve 22 so that the washing liquid can be sprayed for washing the fluorescence dyes remaining on the organism surface after the fluorescence dye mixture has been sprayed and before the fluorescence observation is started. Furthermore, the valve driving circuit 25 switches the valve 22 to the off status after the washing liquid has been sprayed.

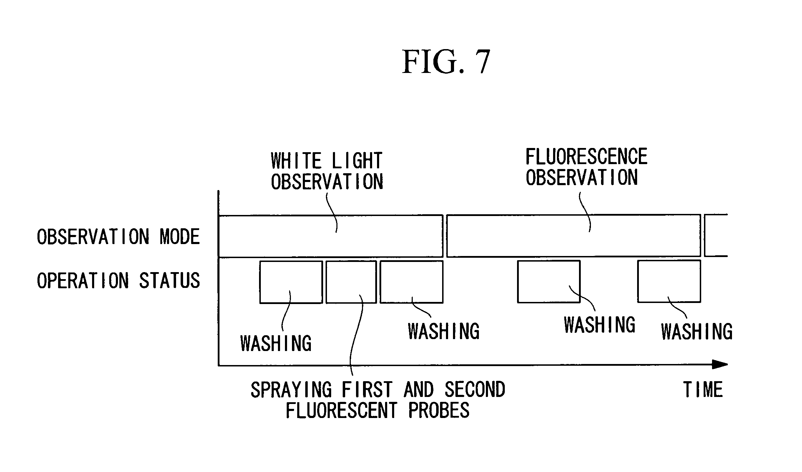

Hereunder is a description of the operation of the thus configured endoscope system 1 according to this embodiment, with reference to FIG. 7.

In order to capture the image of the imaging target A in a body cavity of an organism using the endoscope system 1 according to this embodiment, first, the insertion section 2 is inserted into the body cavity and its distal end 2a is faced to the imaging target A in the body cavity.

In this state, the light source unit 4 and the control unit 5 are operated, and the white observation light source 8 is operated by the operation of the light source controlling circuit 10 to emit light for white light observation to perform the white light observation. At this time, pieces of the reflected light image information acquired by the imaging unit 3 are input into the respective frame memories 17d to 17f of the second storing section 17B, then output to the three R, G, and B channels of the display unit 6, and displayed thereon.

Thereafter, for performing the observation by using the fluorescence dyes, the valve driving circuit 25 operates the valve 22 to connect between the liquid delivery tube 23 and the tank 24 which stores the washing liquid, and thereby the washing liquid is sprayed over the organism to wash out residues remaining on the organism surface.

After the washing liquid has been sprayed, the valve driving circuit 25 operates the valve 22 to connect between the liquid delivery tube 23 and the tank 21 which stores the fluorescence dye mixture, and thereby the fluorescence dye mixture is sprayed over the organism.

After the fluorescence dye mixture has been sprayed, the valve driving circuit 25 operates the valve 22 to connect between the liquid delivery tube 23 and the tank 24 again, and the washing liquid is sprayed to wash out the fluorescence dye mixture remaining on the organism surface. A predetermined time may be allowed from the spraying operation of the fluorescence dyes to the washing operation.

After the washing operation, the light source controlling circuit 10 switches from the white observation light source 8 to the special observation light source 9. First, the variable spectral element driving circuit 16 switches the variable spectral element 13 to the first status to acquire the fluorescence image information Ga of the first fluorescence dye. Next, the variable spectral element is switched to the second status to acquire the fluorescence image information Gb of the second fluorescence dye. Then, the variable spectral element is switched to the first status to acquire the reflected light image information Gc acquired by irradiation of illumination light from the third semiconductor laser.

Then, the image processing circuit 18 receives the fluorescence image information Ga and the fluorescence image information Gb acquired by the irradiation of the excitation lights from the first storing section 17A, performs image processing, calculates the fluorescence image information G1, and outputs the fluorescence image information G1, the fluorescence image information Gb, and the reflected light image information Gc respectively to the R, G, and B channels of the display unit 6. By so doing, the fluorescence image information G1, the fluorescence image information Gb, and the reflected light image information Gc are respectively displayed on the display unit 6.

According to this embodiment, the observation is performed by using the mixture having the first fluorescence dye whose stainability is different between normal tissue and abnormal tissue and the second fluorescence dye whose concentration distribution of adsorption/infiltration is not different between normal tissue and abnormal tissue. Therefore, it becomes possible to acquire the distribution information G1 of the first fluorescence dye after its nonuniformity of the intensity distribution of excitation light on the organism surface has been corrected.

That is to say, the first fluorescence dye is hydrolyzed by a cytoplasmic esterase to change into a fluorescent substance, fluorescein, and then emits intense fluorescence by irradiation of excitation light. This esterase is known to be more abundant in lesioned areas, particularly in cancer tissue, than in normal tissue. For this reason, more intense fluorescence is observed from lesioned areas. On the other hand, the second fluorescence dye is adsorbed/infiltrated at the same concentration in the organism tissue, irrespective of the condition of the tissue, and thus fluorescence is emitted at equal degree of intensity irrespective of the difference between lesioned tissue and normal tissue as long as the excitation light intensity is uniform.

Accordingly, the fluorescence image information Gb from the second fluorescence dye serves as the information which indicates the difference in the intensity of excitation light irradiated on the tissue (distribution/unevenness). As a result, the normalized fluorescence image information G1 in which the nonuniformity of the excitation light intensity and the difference in the adsorptive/infiltrative property of the dye for the organism have been compensated, can be obtained by dividing the fluorescence image Ga from the first fluorescence dye by the fluorescence image Gb from the second fluorescence dye.

That is to say, the fluorescence observation device for organism tissues and the endoscope system 1 according to this embodiment have an advantage in which the concentration distribution of a fluorescence dye that has changed to be fluorescent, is accurately displayed, irrespective of the nonuniformity of the excitation light intensity on the organism tissue being observed, by which normal tissue and abnormal tissue can be discriminated.

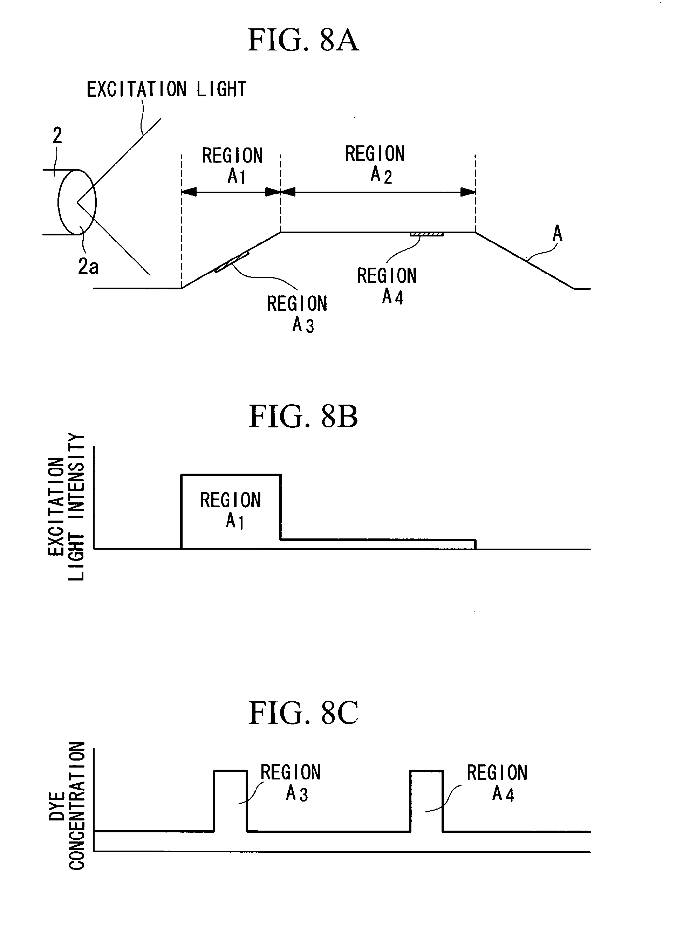

Hereunder is a more conceptual and readily understandable description of the operation of the abovementioned endoscope system 1 according to this embodiment, with reference to FIG. 8A to FIG. 8F.

FIG. 8A is a schematic diagram illustrating the manner of irradiation of excitation light on a rugged organism tissue. The region A1 is a slope opposingly inclined to the surface of the distal end 2a of the insertion section 2, and the region A2 is a flat face approximately parallel to the optical axis of the excitation light. Accordingly, as shown in FIG. 8B, the intensity distribution of excitation light irradiated on the region A is high, whereas the intensity distribution of excitation light irradiated on the region B is low.

Moreover, as shown in FIG. 8C, the region A3 is a region in the region A1 having a high dye concentration, and the region A4 is a region in the region A2 having a high dye region.

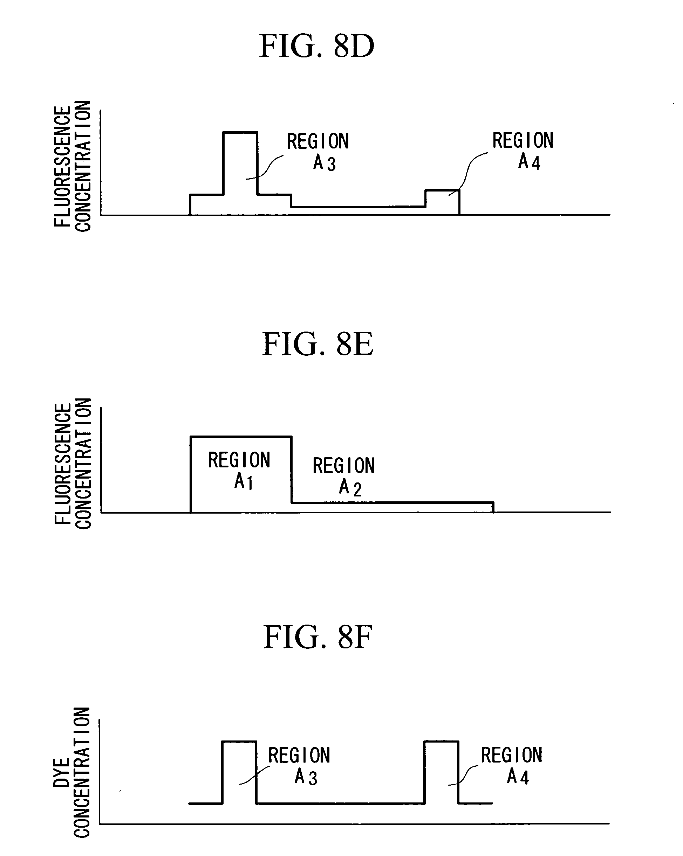

Accordingly, as shown in FIG. 8D, the fluorescence intensity distribution from the first fluorescence dye has a combination pattern of the excitation light intensity distribution and the concentration distribution of the first dye, in which the intensity is high overall in the region A1, particularly high in the region A3, low in the region A2, and slightly higher in the region A4 than in the region A2. For this reason, it is easy to determine that the region A1 is abnormal tissue, whereas it is difficult to determine whether the region A2 is abnormal tissue or not.

On the other hand, as shown in FIG. 8E, the fluorescence intensity distribution from the second fluorescence dye has an approximately same pattern as that of the excitation light intensity distribution.

Here, the information as shown in FIG. 8F in which the excitation light intensity distribution has been corrected so as to reflect the concentration distribution of the first fluorescence dye, can be obtained by dividing the fluorescence intensity distribution from the first fluorescence dye shown in FIG. 8D by the fluorescence intensity distribution from the second fluorescence dye shown in FIG. 8E.

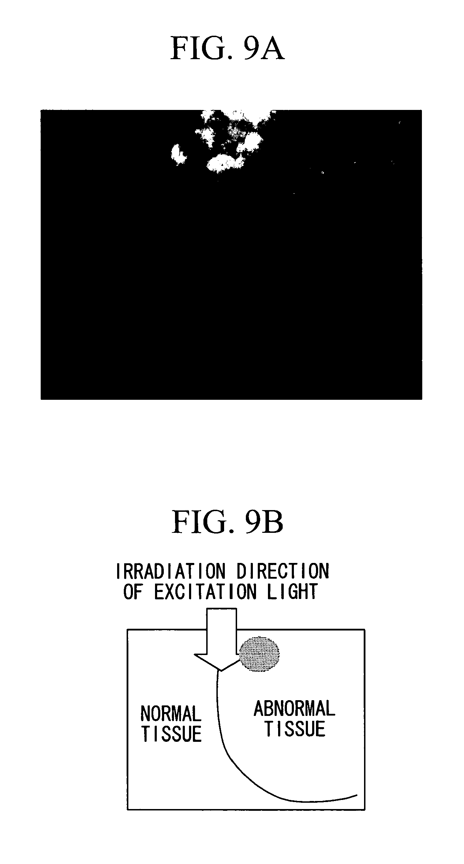

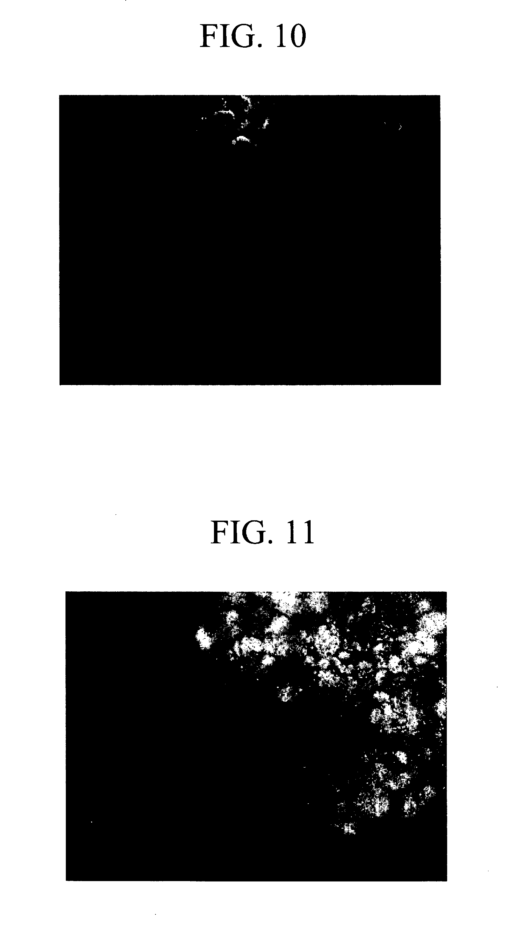

FIG. 9A and FIG. 9B show an example of the fluorescence image information Ga acquired irradiating the first excitation light after the first fluorescent dye has been sprayed. Moreover, FIG. 10 shows an example of the fluorescence image information Gb acquired by irradiating the second excitation light after the second fluorescent dye has been sprayed. Furthermore, FIG. 11 shows an example of the fluorescence image information G1 obtained by dividing the fluorescence image Ga by the fluorescence image Gb.

According to FIG. 9A and FIG. 9B, the fluorescence image information Ga shows the detected fluorescence from the first fluorescent dye that has been accumulated on a cancer tissue, in which higher brightness is obtained in the cancer tissue region than in the normal tissue region. However, highly bright areas do exist only in part of the cancer tissue because of factors such as the arrangement of the light source, the shape of the organism surface, and the like. Therefore, the cancer tissue region is not clear.

Moreover, according to FIG. 10, the fluorescence image information Gb shows overall high brightness since cancer tissue and normal tissue are equally stained. However, similarly to the fluorescence image information Ga, highly bright areas exist in part because of factors such as the arrangement of the light source, the shape of the organism surface, and the like.

Furthermore, according to FIG. 11, the fluorescence image information G1 can clearly show the whole cancer tissue as a highly bright area against the normal site since the fluorescence image information Ga has been normalized by the fluorescence image information Gb.

In this embodiment, a cationic fluorescence dye having a carbocyanine structure is used as the second fluorescence dye. However, another fluorescence dye such as a dye having stainability for the cytoplasmic membrane may also be used as long as its stainability is not different between normal tissue and lesioned tissue and its fluorescence characteristic is different from that of the first fluorescence dye.

In addition, in this embodiment, the fluorescence image information Ga is normalized by using the fluorescence image information Gb to thereby obtain the fluorescence image information G1 in which the nonuniformity of excitation light is eliminated. However, instead of the this procedure, the fluorescence image information Ga and the fluorescence image information Gb may be respectively subjected to image processing to display them on the R and G channels, by which, or by another way, the information of the nonuniformity of excitation light is displayed on the same screen where the fluorescence image information Ga is displayed, so that the observer can recognize the information of the nonuniformity of excitation light. Moreover, modes for respectively displaying the fluorescence image information Ga, the fluorescence image information Gb, and reflected light image information Gc may also be provided.

In this embodiment, the thus calculated fluorescence image information G1, the fluorescence image information Gb, the reflected light image information Gc are displayed. However, if it is desired to display the fluorescence image information Ga, the fluorescence image information Gb, and the reflected light image information Gc in the intact form when acquired, the arrangement may be such that the fluorescence image information Ga, the fluorescence image information Gb, and the reflected light image information Gc are output to the R, G, and B channels of the display unit 6 simply without performing the inter-picture calculation. In addition, the arrangement may be such that the reflected light image information Gc is not displayed while the fluorescence image information Ga and the fluorescence image information Gb are displayed on any of the R, G, and B channels. Moreover, the arrangement may also be such that only the fluorescence image information Ga is output to all of the R, G, and B channels.

In this embodiment, the fluorescence dyes are sprayed immediately after the washing operation, and the fluorescence observation is performed immediately after the re-washing operation. However, the endoscope system 1 may comprise a liquid suction unit (not shown) for sucking the washing liquid, and the fluorescence observation may be performed after the washing liquid puddled on the observation part has been removed by the liquid suction unit.

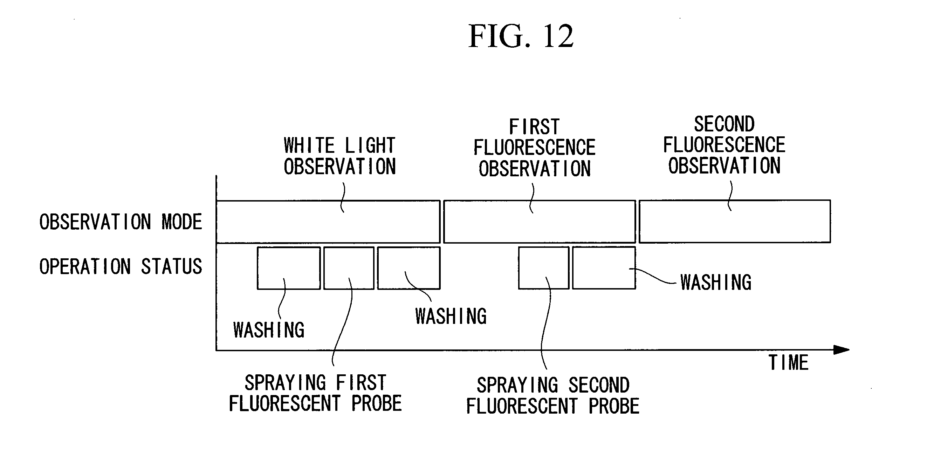

In this embodiment, the mixture having the first fluorescence dye and the second fluorescent dye is sprayed during the white light observation before the light source is switched to perform the fluorescence observation. However, instead of this procedure, as shown in FIG. 12, the first fluorescent dye and the second fluorescent dye may be separately sprayed. In the example shown in the drawing, washing is performed and then the first fluorescent dye is sprayed and washed during the white light observation, in which illumination light is irradiated from the white observation light source. Then, the first excitation light is irradiated from the first semiconductor laser and illumination light is irradiated from the third semiconductor laser to perform the fluorescence observation and the reflected light observation. Next, during this fluorescence observation, the second fluorescent dye is sprayed and washed. Then, the second excitation light is irradiated from the second semiconductor laser to perform the fluorescence observation. The order of these fluorescent dyes may be inverted.

In addition, the arrangement may also be such that washing, spraying and washing of the first fluorescent dye, and spraying and washing of second fluorescent dye are performed during the white light observation, then the fluorescence observation and the reflected light observation are performed by the irradiation of the first excitation light and the illumination light, and the fluorescence observation is performed by the irradiation of the second excitation light.

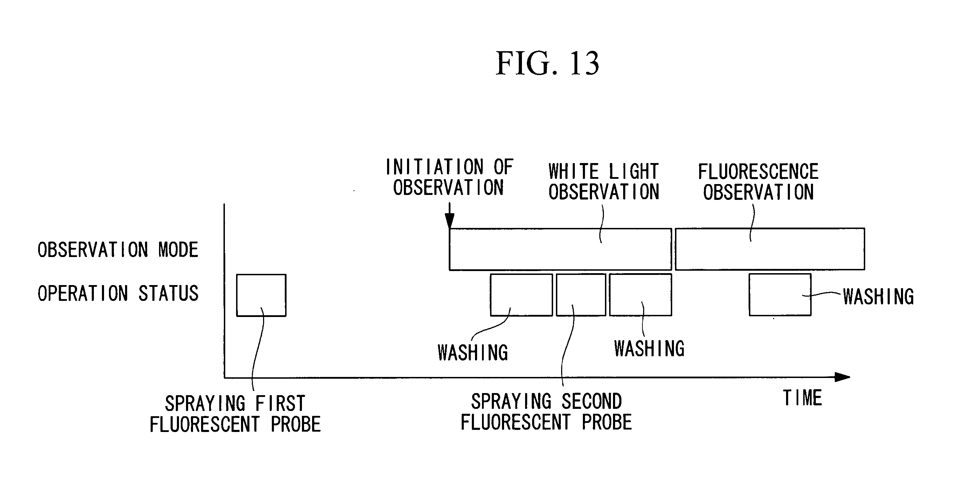

Moreover, since it takes time for the first fluorescent dye to be accumulated on the cancer tissue, then, as shown in FIG. 13, the first fluorescent dye may be previously administered 1 to 24 hours before the observation by means of intravenous injection or oral administration. In this case, an esterase-sensitive fluorescent probe having a fluorescein structure is used as the first fluorescence dye. Furthermore, IR780 is used as the second fluorescent dye (which is sprayed during the observation). In addition, another tumor-affinitive substance such as 5-ALA may also be used as the first fluorescent dye.

**Claims**

1. A fluorescence observation device for organism tissue comprising:

   an excitation light optical system for irradiating an organism tissue adhered or infiltrated with a first fluorescence dye whose stainability is different between normal tissue and abnormal tissue in the organism tissue, and a second fluorescence dye whose fluorescence wavelength or absorption wavelength is different from that of the first fluorescence dye and whose stainability is nonselective between normal tissue and abnormal tissue in the organism tissue, with excitation light which excites the first fluorescence dye and the second fluorescence dye, either simultaneously or in a time sharing manner;
   a fluorescence detecting section for separately detecting fluorescence from the first fluorescence dye and fluorescence from the second fluorescence dye excited by the excitation light from the excitation light optical system;
   a compensation processing section for compensating the fluorescence information from the first fluorescence dye, based on the fluorescence information from the second fluorescence dye detected by the fluorescence detecting section; and
   a display section for displaying the fluorescence information compensated by the compensation processing section.

2. A fluorescence observation device for organism tissue according to claim 1, wherein said second fluorescence dye is cationic.

3. A fluorescence observation device for organism tissue according to claim 1, wherein said second fluorescence dye is lipophilic.

4. A fluorescence observation device for organism tissue according to claim 1, wherein said compensation processing section normalizes the fluorescence information from the first fluorescence dye that has been detected by said fluorescence detecting section, by the fluorescence information from the second fluorescence dye.

5. A fluorescence observation device for organism tissue according to claim 1, wherein said fluorescence detecting section comprises a single observation optical system, whose relative position with respect to said excitation light optical system is fixed, for guiding both fluorescences from the first fluorescence dye and the second fluorescence dye toward said fluorescence detecting section.

FIG. 1

# FIG. 2

## FIG. 3

TRANSMITTANCE

FIRST STATUS OF VARIABLE SPECTRAL ELEMENT

400–560 nm (60% OR GREATER)

FIXED PASSBAND

VARIABLE PASSBAND 610–650 nm (50% OR GREATER)

WAVELENGTH

TRANSMITTANCE

SECOND STATUS OF VARIABLE SPECTRAL ELEMENT

400–560 nm (60% OR GREATER)

FIXED PASSBAND

VARIABLE PASSBAND 780–830 nm (50% OR GREATER)

WAVELENGTH

TRANSMITTANCE

EXCITATION LIGHT CUT FILTER

400–460 nm (80% OR GREATER)

520–720nm (80% OR GREATER)

780–850nm (80% OR GREATER)

480–500 nm (OD VALUE OF 5 OR GREATER)

740–760nm (OD VALUE OF 5 OR GREATER)

WAVELENGTH

INTENSITY

FIRST EXCITATION LIGHT

490±5nm

WAVELENGTH

INTENSITY

SECOND EXCITATION LIGHT

750±5nm WAVELENGTH

INTENSITY

ILLUMINATION LIGHT (IMAGE WHICH REFLECTS BLOOD VOLUME)

420±5nm

WAVELENGTH

INTENSITY

FLUORESCENCE SPECTRUM OF FIRST FLUORESCENCE DYE

WAVELENGTH

INTENSITY

FLUORESCENCE SPECTRUM OF SECOND FLUORESCENCE DYE

WAVELENGTH

INTENSITY

ILLUMINATION LIGHT (WHITE LIGHT OBSERVATION)

B   G   R

WAVELENGTH

# FIG. 4

17

17A

17B

FIRST STORING SECTION

| FRAME MEMORY | FRAME MEMORY | FRAME MEMORY |

17a 17b 17c

SECOND STORING SECTION

| FRAME MEMORY | FRAME MEMORY | FRAME MEMORY |

17d 17e 17f

IMAGING UNIT

17C

EP 2 106 733 A1

# FIG. 5

LIGHT SOURCE   BLUE LIGHT
CONTROLLING   GREEN LIGHT
CIRCUIT   RED LIGHT

VARIABLE   FIRST STATUS
SPECTRAL
ELEMENT   SECOND STATUS

IMAGING
DEVICE

FRAME MEMORY 17d
FRAME MEMORY 17e
FRAME MEMORY 17f

TIME

# FIG. 6

LIGHT SOURCE CONTROLLING CIRCUIT
- FIRST EXCITATION LIGHT
- SECOND EXCITATION LIGHT
- ILLUMINATION LIGHT

VARIABLE SPECTRAL ELEMENT
- FIRST STATUS
- SECOND STATUS

IMAGING DEVICE
- FRAME MEMORY 17a
- FRAME MEMORY 17b
- FRAME MEMORY 17c

TIME

EP 2 106 733 A1

# FIG. 7

WHITE LIGHT OBSERVATION

FLUORESCENCE OBSERVATION

OBSERVATION MODE

OPERATION STATUS

WASHING

SPRAYING FIRST AND SECOND FLUORESCENT PROBES

WASHING

WASHING

WASHING

TIME

# FIG. 8A

EXCITATION LIGHT

2

2a

REGION
A1

REGION
A2

REGION
A3

REGION
A4

A

# FIG. 8B

EXCITATION
LIGHT INTENSITY

REGION
A1

# FIG. 8C

DYE
CONCENTRATION

REGION
A3

REGION
A4

# FIG. 8D

REGION
A3

REGION
A4

FLUORESCENCE
CONCENTRATION

# FIG. 8E

REGION
A1

REGION
A2

FLUORESCENCE
CONCENTRATION

# FIG. 8F

REGION
A3

REGION
A4

DYE
CONCENTRATION

# FIG. 9A

# FIG. 9B

IRRADIATION DIRECTION
OF EXCITATION LIGHT

NORMAL
TISSUE

ABNORMAL
TISSUE

FIG. 10

FIG. 11

# FIG. 12

OBSERVATION MODE — WHITE LIGHT OBSERVATION, FIRST FLUORESCENCE OBSERVATION, SECOND FLUORESCENCE OBSERVATION

OPERATION STATUS — WASHING, SPRAYING FIRST FLUORESCENT PROBE, WASHING, SPRAYING SECOND FLUORESCENT PROBE, WASHING

TIME

EP 2 106 733 A1

# FIG. 13

| | |
|---|---|
| **INTERNATIONAL SEARCH REPORT** | International application No. |
| | PCT/JP2008/051440 |

A. CLASSIFICATION OF SUBJECT MATTER
*A61B1/00*(2006.01)i

According to International Patent Classification (IPC) or to both national classification and IPC

B. FIELDS SEARCHED

Minimum documentation searched (classification system followed by classification symbols)
A61B1/00

Documentation searched other than minimum documentation to the extent that such documents are included in the fields searched
Jitsuyo Shinan Koho          1922-1996    Jitsuyo Shinan Toroku Koho   1996-2008
Kokai Jitsuyo Shinan Koho    1971-2008    Toroku Jitsuyo Shinan Koho   1994-2008

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 9-294706 A  (Fuji Photo Film Co., Ltd.), 18 November, 1997 (18.11.97), (Family: none) | 1-5 |
| A | JP 2006-175052 A  (Fuji Photo Film Co., Ltd.), 06 July, 2006 (06.07.06), (Family: none) | 1-5 |
| A | JP 2004-478 A  (Fuji Photo Film Co., Ltd.), 08 January, 2004 (08.01.04), & US 2003/216626 A1 | 1-5 |

☐  Further documents are listed in the continuation of Box C.          ☐  See patent family annex.

| | |
|---|---|
| *     Special categories of cited documents: | "T"   later document published after the international filing date or priority date and not in conflict with the application but cited to understand the principle or theory underlying the invention |
| "A"   document defining the general state of the art which is not considered   to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| | |
|---|---|
| Date of the actual completion of the international search | Date of mailing of the international search report |
| 14 February, 2008 (14.02.08) | 26 February, 2008 (26.02.08) |
| Name and mailing address of the ISA/ | Authorized officer |
| Japanese Patent Office | |
| Facsimile No. | Telephone No. |

Form PCT/ISA/210 (second sheet) (April 2007)

**EP 2 106 733 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI10201707 B

- JP HEI627110 B